# EUROPEAN PATENT SPECIFICATION

(11) **EP 0 674 896 B1**
(45) Date of publication and mention of the grant of the patent: **22.09.1999**
(21) Application number: 94830479.5
(22) Date of filing: 07.10.1994
(51) Int. Cl.: A61K 6/083

(54) **Synthetic resin-based dental material composition with antiseptic properties**
Dentalmasse auf der Basis von Kunstharzen, mit antiseptischen Eigenschaften
Matériau dentaire à base de composition de résins synthétique ayant des propriétés antiseptiques

(30) Priority: 28.03.1994 IT RM940172
(43) Date of publication of application: 04.10.1995
(73) Proprietor: BANDINI s.r.L, I-00155 ROMA (IT)
(72) Inventor: Bandini, Silvio, I-00155 Roma (IT)
(74) Representative: Banchetti, Marina

(56) References cited:
- EP-A- 0 264 660
- WO-A-93/08792
- WO-A-94/18939
- GB-A- 2 193 967
- US-A- 5 080 583

## Description

The present invention relates to a synthetic resin-based dental material composition with antiseptic properties. More particularly, the invention concerns synthetic polymer materials for use in dental restoration and for the production of false teeth, bridges and dental prostheses, which materials are endowed with inherent germicidal and cariostatic properties.

As it is known, tooth crowns, bridges and partial or complete dentures used to be made in the past either of metallic materials such as gold, silver and suitable alloys thereof with other metals, or of ceramic materials such as feldspar or quartz. In order to exploit at one time the good mechanical properties of the metal and the superior appearance of the ceramic material, which can closely reproduce the colour and brightness of natural enamel, metal-ceramic combinations are presently used. They consist of a metallic structure coated with a thin layer of a suitable ceramic material. However, these combined materials have the drawback of chipping easily, owing to the low tensile strength of the outer ceramic layer.

More recently, synthetic resins have been developed for use as dental materials, and they are now employed not only for restorative fillings and for the restoration of partially damaged teeth, but also for the production of artificial denture bases, of dental crowns, of fixed and removable bridges and dentures. The synthetic materials most widely used in this field are the acrylic resins, more specifically the resins based on poly(methyl methacrylate), which show a good compatibility with human tissues, good mechanical properties, absence of particular taste or odour once polymerised, low water absorption, good shape stability as well as ability to exactly reproduce shape details, colour stability and acceptable aesthetic appearance, ease of repairing and cleaning.

In order to reduce the shrinkage which occurs during polymerisation of methyl methacrylate, thus reducing at the same time the heat release, the most employed production technique consists in admixing about three parts of acrylic polymer with one part of the methyl methacrylate monomer, so as to obtain a formable dough. The complete polymerisation of the latter is achieved by means of free-radical initiators, such as organic peroxides (e.g., benzoyl peroxide), or by means of photo-initiators, such as some aromatic ketones (e.g., camphoroquinone), in co-operation with UV or visible light.

When polymerisation is started by free-radical initiators the dental material consists of two separate components, one of which contains the polymerisable material while the other contains the catalyst. For heat-cured polymers only a free-radical initiator such as benzoyl peroxide is present as the catalyst component, while for cold-cured polymers an additional ingredient, known as the "activator", is present in the polymerisable mixture. The activator, usually a tertiary amine such as N,N-dimethyl-p-toluidine, forms a redox system with the free-radical initiator contained in the catalyst component, which redox system starts polymerisation at room temperature.

When polymerisation is started by the action of light the polymerisable dental material is a single-component mixture containing one or more photo-initiators together with the polymerisable ingredients.

The synthetic resin-based mixtures for use as dental materials may also contain additional ingredients such as, e.g., cross-linking agents (e.g. ethylene glycol dimethacrylate) and finely divided inorganic material (i.e. the inert fillers), intended to provide the desired colour and mechanical properties, such as hardness, wear resistance and resistance to abrasion.

Besides the acrylic resins, some polycarbonates, resins based on poly(vinyl chloride) (specifically, copolymers of vinyl chloride, vinyl acetate and methyl methacrylate), some polyamides and some polyurethanes have been tested for their performance as denture base materials and the like, but the results reported have been, so far, not entirely satisfactory.

As concerns the specific field of restorative fillings, the use of poly(methyl methacrylate) resins has been discontinued in favour of specially designed materials whose main monomer component is the so-called Bowen monomer or bis-GMA (or BISGMA, bisphenol glycidyl methacrylate), i.e. the diester of bisphenol A di(2,3-epoxy propyl)ether with methacrylic acid. Normally, mixtures of BISGMA with diluent monomers such as triethylene glycol dimethacrylate (also known as TEGDMA) are used.

Also in this case, the material comprises inorganic fillers and may contain a number of further additives. The polymerisation may either be started by a free-radical initiator at room temperature (cold-cured systems, also known as paste-paste systems) or by the action of UV or visible light (light-cured systems, also known as single component systems).

One of the most critical problems encountered in the use of artificial dental elements is that of maintaining a proper oral hygiene. When artificial dental crowns and bridges are present, the gums tend to retract, thus uncovering the gingival groove and the tooth neck, where the bacterial flora more easily finds the optimal conditions for its development. This is also due to the frequent presence in the said zone of food residues, which escape the usual cleaning. As its known, the foregoing on one hand co-operates in giving rise to problems of unpleasant breath and, on the other hand, may result in paradental inflammations, such as, specifically, gingivitis.

Further, bacterial plaque on natural teeth normally favours the formation of dental cares. The organic acids which are produced by the action of the oral bacteria decalcify the hard tissues of the teeth, thus giving rise to the formation of small cavities wherein the micro-organisms find conditions favourable to their growth. Although dentistry is nowadays provided with means for stopping the caries' progress, by destroying the affected tissue starting from the exterior surface towards the interior of the tooth, no effective and readily applicable means have been found up to now for exerting a preventive action against caries, substantially owing to the fact that the knowledge on the etiopathogenesis of this affection is still incomplete.

Besides representing a danger for natural, still undamaged teeth, dental cares may also challenge teeth that have already been treated and restored by means of suitable fillings. In spite of the fact that the restoration of a tooth partially damaged by caries is carried out with the utmost care, it may well occur that cares newly develops under the restorative filling, and that the latter is finally lost as a result of the caries' progress.

Therefore, it is an object of the present invention to provide dental material compositions suitable for the production of dental prostheses, false teeth, bridges and the like, as well as for use as restorative fillings, which show, in addition to the necessary mechanical and aesthetic features mentioned above, the further property of having a long-lasting antiseptic and germicidal action, so as to hinder the growth of bacterial plaque and prevent the caries formation.

In accordance with the present invention there is suggested to incorporate, in a resin-based formulation of the kind suitable for the production of dental elements or fillings, a mixture of essential oils having germicidal and bacteriostatic activity, as well as a quaternary ammonium compound, also with the function of antiseptic.

It is to be noted that a dental element having inherent germicidal activity could not be obtained, e.g., by employing the conventional ceramic materials, since the latter are subjected during production to temperatures so high that the bactericidal organic compounds mentioned above would surely decompose, thus losing any biological activity.

Therefore, the present invention specifically provides a synthetic resin-based dental material composition characterised in that it also comprises a quaternary ammonium compound having germicidal and/or bacteriostatic activity and a mixture of essential oils comprising mint oil, eucalyptus oil and bergamot oil.

Preferably, said synthetic resin is chosen from the group consisting of acrylic polymers or copolymers (e.g. poly(methyl methacrylate)-based polymers), unsaturated polyester resins, vinyl ester resins, and polymers or copolymers of BISGMA (e.g. copolymers of BISGMA and TEGDMA). Advantageously, said dental material may comprise one or more inorganic fillers chosen from the group consisting of glass micro-beads, quartz particles, corundum particles, micronized silica, fumed silica.

In the present disclosure there is meant by "dental material" any material suitable for the production of partial or complete dentures, fixed or removable dental bridges, crowns and the like, as well as any material suitable for being used in dentistry as binding or sealant resin, and for carrying out partial or total restorations of either natural or artificial teeth, such as materials for restorative fillings. Any such element will be also referred to in the following as "dental element".

The quaternary ammonium compound which according to the invention is added to the dental material formulation is, advantageously, a quaternary ammonium salt wherein at least one of the four groups bound to the nitrogen atom is a long-chain alkyl group. As a matter of fact, it is known that this class of compounds comprises several products endowed with antibacterial, antifungal and generally disinfecting activity which show an extremely reduced toxicity towards higher organisms. Particularly common examples of such compounds are dialkyl-dimethyl ammonium salts (such as, e.g., didecyl-dimethyl ammonium chloride), cetylpyridinium chloride and alklyl-dimethyl-benzyl ammonium salts, including, first of all, benzalkonium chloride. More precisely, the latter is a mixture of alkyl-dimethyl-benzyl ammonium chlorides whose alkyl chains comprise between 8 and 18 carbon atoms. Benzalkonium chloride is known for having a high germicidal activity, even at low concentrations, towards a wide range of micro-organisms, including Gram-positive and Gram-negative bacteria.

In the formulation according to the instant invention the disinfecting action of the quaternary ammonium compound is synergically supported by the essential oils. Although some of said essential oils have been known to possess disinfecting and antimicrobial activity since ancient times, the fact that said activity is effectively and lastingly exerted when the product is incorporated in a solid and coherent mixture such as that making out a dental element, without negatively affecting, on the other hand, the mechanical properties of said dental element, does not appear to have been disclosed before, and is a specific aspect of this invention.

As it is known, essential oil or essences are active ingredients of vegetable origin which are contained in a great number of plants, from which they can be obtained by pressing or by distillation with water steam or by solvent extraction. The products obtained by extractive means such as solvents or liquid or semiliquid fats are more properly called extracts.

Among the essential oils, mint oil is obtained by steam distillation of the leaves and the flowering tips of the Mentha piperita plant, and is a clear colourless or yellowish or some times greenish liquid, whose typical burning odour is followed by a freshness feeling. The essence mainly contains (-)-menthol as the active ingredient, and is used for flavouring toothpastes, confectioneries and chewing gums.

The eucalyptus oils are obtained from the leaves of various plants belonging to the genus Eucalyptus, among which, firstly, Eucalyptus globulus. The extract of the latter, having a characteristic fresh odour and a colourless or pale yellow aspect, is known as eucalyptole, and contains cineole and α-pinene. Said product is employed in pharmaceutical preparations as an antiseptic for the respiratory tract.

The bergamot oil is obtained by pressing peels from the Citrus aurantium fruit, and is a brownish yellow or greenish yellow liquid, which sometimes contains a deposit. The odour is pleasant, fruity, fresh and sweet. The most important components of bergamot oil are linalyl acetate, linalol and citral, besides the terpene compounds. In addition to being used in perfume production and in cosmetic industry, bergamot oil is also employed in medicinal products, owing to its topical antiseptic properties.

In addition to the final (i.e., already polymerised) dental material compositions mentioned above, this invention also concerns the formulations starting from which said dental material is obtained, i.e., the compositions before the polymerisation step. Specifically, the invention provides compositions for the production of synthetic resin-based dental elements, which compositions may either be of the two-components type, or of the single-component type.

In the former case, as usual, the first component comprises a polymerisable synthetic product and the second component comprises a free-radical polymerisation initiator, and the composition is characterised in that the first component also comprises a quaternary ammonium compound having germicidal and/or bacteriostatic activity and a mixture of essential oils comprising mint oil, eucalyptus oil and bergamot oil.

In accordance with what set forth before as concerns the preferred synthetic resins for use in the proposed formulations, said polymerisable synthetic product is preferably chosen from the group consisting of acrylic and/or methacrylic compounds, mixtures of unsaturated and saturated carboxylic acids with diols (which give rise by polymerisation to unsaturated polyester resins), mixtures of liquid epoxy resins with acrylic acid (which give rise by polymerisation to vinyl ester resins), BISGMA (i.e. bisphenol glycidyl methacrylate) and mixtures thereof with other dimethacrylates.

The inorganic fillers referred to before may be contained either in said first component or in said second component or in both, as it will be shown in the following examples of two-components formulations according to the invention. All percentage amounts given below are by weight.

### EXAMPLE 1

| Two-components formulation to give poly(methyl methacrylate)-based polymer · Component 1 | |
|---|---|
| mixture of poly(methyl methacrylate) and methyl methacrylate | qs. to 100% |
| activator (N,N-dimethyl-p-toluidine) | 0.7-1% |
| mixture of essential oils (mint oil, eucalyptus oil and bergamot oil) | 1.2-1.5% |
| benzalkonium chloride | 2.4-3.0% |

| · Component 2 | |
|---|---|
| benzoyl peroxide | 1.7-2.0% |
| micronized silica | 0.9-1.2% |
| glass micro-beads | qs. to 100% |

### EXAMPLE 2

| Two-components formulation to give poly(methyl methacrylate)-based polymer · Component 1 | |
|---|---|
| methyl methacrylate containing 6% of cross-linking agent (ethylene glycol dimethacrylate) | 97-97.9% |
| activator (N,N-dimethyl-p-toluidine) | 0.7-1% |
| mixture of essential oils (mint oil, eucalyptus oil and bergamot oil) | 1.2-1.5% |
| benzalkonium chloride | 0.2-0.5% |

| · Component 2 | |
|---|---|
| poly(methyl methacrylate) | qs. to 100% |
| benzoyl peroxide | 0.3-3.1% |
| quartz or corundum particles | 8-10% |
| glass micro-beads | 8-10% |
| inorganic pigments for various shades | 0.8-1.5% |

The above formulation is intended for use in the production of temporary dental prostheses and bridges. About 50% of the whole mass of inert fillers has particle size comprised between 13 and 20 µm. As is usual in the production of dental materials, the inert fillers are previously treated with about 0.025-2% of methacryloxypropyltrimethoxysilane in order to enhance the matrix-to-filler bonding.

### EXAMPLE 3

| Two-components formulation to give unsaturated polyester resins or vinyl ester resins · Component 1 | |
|---|---|
| mixture of unsaturated and saturated carboxylic acids with diols, or mixture of liquid epoxy resins with acrylic acid | qs. to 100% |
| cross-linking agent (stirene) | 2.4-3.0% |
| activator (cobalt octoate) | 0.1-0.5% |
| deaerating agent | 0.12-0.15% |
| mixture of essential oils (mint oil, eucalyptus oil and bergamot oil) | 0.2-0.3% |
| benzalkonium chloride | 0.15-0.22% |
| glass micro-beads | 29-35% |
| micronized silica | 0.7-0.9% |

| · Component 2 | |
|---|---|
| methyl ethyl ketone peroxide, amount referred to component 1 | 2-3% |

### EXAMPLE 4

| Two-components formulation with BISGMA and TEGDMA (paste-paste system for dental restorations) · Component 1 | |
|---|---|
| BISGMA (75%) and TEGDMA (25%) | qs. to 100% |
| stabiliser (hydroquinone) | 0.1% |
| activator (N,N-dimethyl-p-toluidine) | 0.7-1% |
| mixture of essential oils (mint oil, eucalyptus oil and bergamot oil) | 1.2-1.5% |
| benzalkonium chloride | 0.2-0.5% |
| quartz or corundum particles | 30% |
| glass micro-beads | 28% |
| fumed silica | 10% |

| · Component 2 | |
|---|---|
| BISGMA (75%) and TEGDMA (25%) | qs. to 100% |
| benzoyl peroxide | 0.3-3.1% |
| quartz or corundum particles | 30% |
| glass micro-beads | 28% |
| fumed silica | 11.7% |

Also in this case the inert fillers are previously treated with about 0.025-2% of methacryloxypropyltrimethoxysilane in order to enhance the matrix-to-filler bonding.

The compositions of the single component type according to the invention comprise, as it is customary, a polymerisable synthetic product and a photo-initiator system, and are characterised in that said single component also comprises a quaternary ammonium compound having germicidal and/or bacteriostatic activity and a mixture of essential oils comprising mint oil, eucalyptus oil and bergamot oil.

Preferably, said polymerisable synthetic product is chosen from the group consisting of acrylic and/or methacrylic compounds, BISGMA (i.e. bisphenol glycidyl methacrylate) and mixtures thereof with other dimethacrylates.

Light-cured dental compositions intended for being polymerised by the action of UV light generally contain, as the initiator, an aromatic ketone (such as 1,2-diphenyl-2,2-dimethoxy ethanone and 2-methoxy-2-phenyl acetophenone, also known as benzoin-methyl ether), while compositions which may be cured by the action of visible light generally contain a photo-initiator system with two ingredients, generally camphoroquinone and an aliphatic or aromatic amine (such as a N,N-aminoethyl methacrylate), the latter serving as a polymerisation promoter.

Examples of single-component composition according to the invention, for use as restorative filling, are as follows. All percentage amounts given below are by weight.

### EXAMPLE 5

| Single-component formulation with BISGMA and TEGDMA (UV light-cured composite for dental restorations) · Composition | |
|---|---|
| BISGMA (75%) and TEGDMA (25%) | qs. to 100% |
| stabiliser (benzophenone) | 0.1% |
| benzoin-methyl ether | 0,2% |
| mixture of essential oils (mint oil, eucalyptus oil and bergamot oil) | 1.2-1.5% |
| benzalkonium chloride | 0.2-0.5% |
| quartz or corundum particles | 30% |
| glass micro-beads | 28% |
| fumed silica | 10% |

### EXAMPLE 6

| Single-component formulation with BISGMA and TEGDMA (visible light-cured composite for dental restorations) · Composition | |
|---|---|
| BISGMA (75%) and TEGDMA (25%) | qs. to 100% |
| stabiliser (benzophenone) | 0.1% |
| camphoroquinone | 0,2% |
| N,N-aminoethyl methacrylate | 0,1% |
| mixture of essential oils (mint oil, eucalyptus oil and bergamot oil) | 1.2-1.5% |
| benzalkonium chloride | 0.2-0.5% |
| quartz or corundum particles | 30% |
| glass micro-beads | 28% |
| fumed silica | 10% |

As pointed out, the formulations disclosed in examples 5 and 6 are suitable for use, respectively, with UV light sources and with sources irradiating visible light. It is also possible, in order to avoid the need of differentiated restorative filling products depending on the light source available to the dentist, to provide a product according to the invention which includes in one only formulation both initiator systems.

The production techniques of dental elements by employing the formulations according to this invention are not substantially different from those known in the art. The only specific aspect that should be taken into account is the limited resistance of the organic antiseptic compounds of the invention to severe temperature conditions. For instance, in the production of acrylic resin temporary prostheses the exothermic polymerisation reaction brings about an increase in the reaction mixture temperature, which rapidly reaches a peak level of 38-40 °C, and then slowly returns to room levels. It is important, as pointed out before, that the exothermic peak does not exceed the said values.

By way of an example, the results of some experimentations carried out on samples of antiseptic dental material according to the invention are set forth below. The active materials subjected to the tests have been obtained from the polymerisation of two components formulated as follows.

| · Component 1 | |
|---|---|
| mixture of poly(methyl methacrylate) and methyl methacrylate | 250 g |
| mixture of essential oils (mint oil, eucalyptus oil and bergamot oil) | 3.54 g |
| benzalkonium chloride | 7.10 g |

| · Component 2 | |
|---|---|
| glass micro-beads (62-200 µm) | 375 g |
| benzoyl peroxide | 7.50 g |
| micronized silica | 4.16 g |

Said material has been used to produce round plates 4 cm in diameter and some millimetres in thickness. The control plates, of the same shape and size, only contain the resin and the inert fillers. The features of four of said sample plates are as follows:
- sample No. 1: control
- sample No. 2: control
- sample No. 3: composition according to the invention produced some days before the test
- sample No. 4: composition according to the invention produced more that 1 year before the test

In order to evaluate the action of the above samples on micro-organisms, bacterial strains have been used which had been isolated from clinical materials (more precisely, oropharyngeal exudates) of out-patients. The bacterial species examined are the following:
alpha-haemolytic streptococci;
Staphylococcus epidermidis;
beta-haemolytic streptococci;
Staphylococcus aureus;
Pseudomonas aeruginosa;
Klebsiella pneumoniae;
Proteus mirabilis.

All of the strains have been isolated on solid media and have been identified through their morphological and cultural characteristics, and by means of biochemical tests. From all of the strains suspensions in sterile distilled water have been prepared, so as to obtain a turbidity of 7.5 x 10⁶ cells/ml as measured on the McFarland turbidimetric scale. After dividing each plate into 7 circular sectors, each one of the bacterial suspensions prepared has been placed in one sector of each plate, by means of a 10 µl graduated pipette. The samples so treated have been left for 4 hours closed in sterile Petri dishes, on the bottom of which some blotting paper impregnated with sterile distillated water had been placed in order to moisturise the interior of the dishes. The temperature was the room temperature, oscillating between 18 °C and 20 °C.

After 4 hours some sterile swabs were passed over each sector of the plates, which swabs were dissolved in 22 ml of tripton soya broth. The resulting mixture was incubated at 37 °C for 24 hours. Subsequently, the broth cultures were used for seeding on selective means, in order to identify the micro-organisms. The culture means employed were the following:
alpha-haemolytic streptococci: modified CNA Columbia agar-blood;
Staphylococcus epidermidis: mannite salt agar;
beta-haemolytic streptococci: modified CNA Columbia agar-blood;
Staphylococcus aureus: mannite salt agar;
Pseudomonas aeruginosa: cetrimide agar;
Klebsiella pneumoniae: McConkey agar;
Proteus mirabilis: McConkey agar.

The results obtained from the above experimental procedure are set forth in the following table, wherein "-" represents no micro-organisms growth, "+" represents a somewhat limited growth, "++" and "+++" respectively represent a good growth and a particularly abundant growth.

**TABLE 1**

| Micro-organisms growth on acrylic resin-based plates | | | | |
|---|---|---|---|---|
| MICRO-ORGANISM | SAMPLE No. | | | |
| | 1 | 2 | 3 | 4 |
| alpha-haemolytic streptococci | +++ | + | - | - |
| Staphylococcus epidermidis | +++ | + | - | - |
| beta-haemolytic streptococci | +++ | - | - | - |
| Staphylococcus aureus | +++ | - | - | - |
| Pseudomonas aeruginosa | +++ | ++ | - | - |
| Klebsiella pneumoniae | +++ | ++ | - | - |
| Proteus mirabilis | +++ | ++ | - | - |

The results reported above confirm the germicidal activity of the resin-based compositions according to the invention. In fact, both in sample 3 and in sample 4 benzalkonium chloride and the essential oils have been shown to greatly interfere with the growth of the bacterial strains placed on the sample itself: none of the seedings carried out on each sector of the examined plates appears to have resulted in the growth any micro-organism, either Gram-positive or Gram-negative.

On the contrary, on all of the sectors of the control plate No. 1 an abundant bacterial growth took place. As far as the control plate No. 2 is concerned, the growth of the three Gram-negative species (i.e., Ps. aeruginosa, K. pneumoniae and P. mirabilis), of a quite low number of colonies of alpha-haemolytic streptococci and of Staphylococcus epidermidis was detected, while beta-haemolytic streptococci and Staphylococcus aureus did not develop at all. It is to be noted that before the test was carried out the sample No. 2 had been left for some time into contact with one of the samples of the material according to the invention, since the plates had been kept stacked one over the other; the fact that the same microbial strains, in the same conditions, abundantly grew on plate No. 1 but not as much on plate No. 2 shows that the simple contact with the proposed germicidal material may negatively affect the bacterial growth even on substrates which have not the composition according to the invention. This allows to foresee that the presence in the oral cavity of a dental element according to the invention may exert a preventive anti-caries action towards the natural teeth located close to said dental element.

Coming back to samples 3 and 4, made of the proposed dental material, it is worthwhile noting that the sample No. 4, which had been produced more than one year before the test, still showed the same activity level as the freshly made sample. The foregoing is an evidence of the fact that the antiseptic activity of the materials according to the invention lasts for a long time. Such property is clearly fundamental in view of the average life time required to dental elements such as bridges, crowns, prostheses and restorative fillings.

## Claims

1. Synthetic resin-based dental material composition, characterised in that it also comprises a quaternary ammonium compound having germicidal and/or bacteriostatic activity and a mixture of essential oils comprising mint oil, eucalyptus oil and bergamot oil.

2. Composition according to claim 1 wherein said synthetic resin is chosen from the group consisting of: acrylic polymers or copolymers, unsaturated polyester resins, vinyl ester resins, polymers or copolymers of BISGMA (i.e. bisphenol glycidyl methacrylate).

3. Composition according to claim 2 wherein said acrylic polymer or copolymer is a poly(methyl methacrylate)-based polymer.

4. Composition according to claim 2 wherein said polymer or copolymer of BISGMA is a copolymer of BISGMA and TEGDMA (i.e. triethylene glycol dimethacrylate).

5. Composition according to any one of claims 1-4, wherein said quaternary ammonium compound is benzalkonium chloride.

6. Composition according to any one of claims 1-5, also comprising one or more inorganic fillers chosen from the group consisting of: glass micro-beads, quartz particles, corundum particles, micronized silica, fumed silica.

7. Composition for the production of synthetic resin-based dental elements, said composition being of the two-components type, wherein the first component comprises a polymerisable synthetic product and the second component comprises a free-radical polymerisation initiator, characterised in that said first component also comprises a quaternary ammonium compound having germicidal and/or bacteriostatic activity and a mixture of essential oils comprising mint oil, eucalyptus oil and bergamot oil.

8. Composition according to claim 7, wherein said polymerisable synthetic product is chosen from the group consisting of: acrylic and/or methacrylic compounds, mixtures of unsaturated and saturated carboxylic acids with diols, mixtures of liquid epoxy resins with acrylic acid, BISGMA (i.e. bisphenol glycidyl methacrylate) and mixtures thereof with other dimethacrylates.

9. Composition according to any one of claims 7-8 wherein said quaternary ammonium compound is benzalkonium chloride.

10. Composition according to any one of claims 7-9 wherein either said first component or said second component or both also comprise one or more inorganic fillers chosen from the group consisting of: glass micro-beads, quartz particles, corundum particles, micronized silica, fumed silica.

11. Composition according to claim 10 wherein said first component comprises a mixture of poly(methyl methacrylate) and methyl methacrylate, benzalkonium chloride, a mixture of essential oils comprising mint oil, eucalyptus oil and bergamot oil and a polymerisation activator, and said second component comprises benzoyl peroxide, glass micro-beads and micronized silica.

12. Composition according to claim 10 wherein said first component comprises methyl methacrylate, benzalkonium chloride, a mixture of essential oils comprising mint oil, eucalyptus oil and bergamot oil, a cross-linking agent and a polymerisation activator, and said second component comprises poly(methyl methacrylate), benzoyl peroxide, glass micro-beads and quartz or corundum particles.

13. Composition according to claim 10 wherein said first component comprises a mixture of unsaturated and saturated carboxylic acids with diols, or a mixture of liquid epoxy resins with acrylic acid, benzalkonium chloride, a mixture of essential oils comprising mint oil, eucalyptus oil and bergamot oil, glass micro-beads, micronized silica, a cross-linking agent and a polymerisation activator, and said second component comprises methyl ethyl ketone peroxide.

14. Composition according to claim 10 wherein said first component comprises a mixture BISGMA (i.e. bisphenol glycidyl methacrylate) and TEGDMA (i.e. triethylene glycol dimethacrylate), benzalkonium chloride, a mixture of essential oils comprising mint oil, eucalyptus oil and bergamot oil, quartz or corundum particles, glass micro-beads, fumed silica and a polymerisation activator, and said second component comprises a mixture of BISGMA (i.e. bisphenol glycidyl methacrylate) and TEGDMA (i.e. triethylene glycol dimethacrylate), benzoyl peroxide, quartz or corundum particles, glass micro-beads, and fumed silica.

15. Composition for the production of synthetic resin-based dental elements, said composition being of the single component type, wherein said single component comprises a polymerisable synthetic product and a photo-initiator system, characterised in that said single component also comprises a quaternary ammonium compound having germicidal and/or bacteriostatic activity and a mixture of essential oils comprising mint oil, eucalyptus oil and bergamot oil.

16. Composition according to claim 15, wherein said polymerisable synthetic product is chosen from the group consisting of: acrylic and/or methacrylic compounds, BISGMA (i.e. bisphenol glycidyl methacrylate) and mixtures thereof with other dimethacrylates.

17. Composition according to any one of claims 15-16 wherein said quaternary ammonium compound is benzalkonium chloride.

18. Composition according to any one of claims 15-17 wherein said single component also comprises one or more inorganic fillers chosen from the group consisting of: glass micro-beads, quartz particles, corundum particles, micronized silica, fumed silica.

19. Composition according to claim 18 wherein said single component comprises a mixture of BISGMA (i.e. bisphenol glycidyl methacrylate) and TEGDMA (i.e. triethylene glycol dimethacrylate), benzalkonium chloride, a mixture of essential oils comprising mint oil, eucalyptus oil and bergamot oil, quartz or corundum particles, glass micro-beads, fumed silica and a photo-initiator system.

## Patentansprüche

1. Dentalmasse aus Kunstharzen, dadurch gekennzeichnet, dass sie auch eine Quarternär-Ammoniumverbindung enthält, die eine keimtötende und bakteriostatische Wirkung hat und ein Gemisch von ätherischen Ölen mit Minzöl, Eukalyptusöl und Bergamotöl enthält.

2. Dentalmasse aus Kunstharzen nach Anspruch 1, worin das vorgenannte Kunstharz aus einer Gruppe ausgewählt ist, die aus Akryl-Polymeren oder Copolymeren, ungesättigten Polyester-Harzen, Vinylester-Harzen, Polymeren oder Copolymeren von BISGMA (d.h. Bisphenol-Glycidyl-Methacrylate) besteht.

3. Dentalmasse aus Kunstharzen nach Anspruch 2, worin der vorgenannte Polymer oder Copolymer ein Poly(Methyl-Methacrylat)-Polymer ist.

4. Dentalmasse aus Kunstharzen nach Anspruch 2, worin der vorgenannte Polymer oder Copolymer von BISGMA ein Copolymer von BISGMA und TEGDMA (d. h. Triäthylen-Glycol-Dimethylacrilat) ist.

5. Dentalmasse aus Kunstharzen nach je einem der Ansprüche 1 bis 4, worin die vorgenannte Quarternär-Ammoniumverbindung Benzalkonium-Chlorid ist.

6. Dentalmasse aus Kunstharzen nach je einem der Ansprüche 1 bis 5, die ausserdem ein oder mehrere Füllmittel enthält, die aus einer Gruppe ausgewählt sind, welche aus Glass-Mikrokugelchen, Quarzpartikeln, Korundpartikeln, Mikrokiesel, Rauchkiesel besteht.

7. Verbindung zur Herstellung von Kunstharz-Dentalteilen, wobei die vorgenannte Verbindung der zwei Bestandteile enthaltenden art ist, worin der erste Bestandteil ein polymerisierbarer Kunststoff ist und der zweite Bestandteil ein Initiator von Frei-Radikal-Polymerisation ist, dadurch gekennzeichnet, dass der vorgenannte erste Bestandteil gleichfalls eine Quarternär-Ammoniumverbindung enthält, die eine keimtötende und/oder bakteriostatische Wirkung aufweist und ein Gemisch von ätherischen Ölen mit Minzöl, Eukaliptusöl und Bergamotöl enthält.

8. Verbindung nach Anspruch 7, worin der vorgenanten polymerisierbare kunststoff aus akrylischen und/oder methakrylischen Verbindungen, Gemischen von ungesättigten und gesättigten Karbonsäuren mit Diolen, Mischungen von flüssigen Epoxyharzen mit Akrylsäure, BISGMA (Bisphenol-Glycidil-Methakrilat) und Mischungen von anderen Dimethakrylaten besteht.

9. Verbindung nach je einem der Ansprüchen 7 und 8, worin die vorgenannte Quarternär-Ammoniumverbindung Benzakonium-Chlorid ist.

10. Verbindung nach je einem der Ansprüchen 7 und 8, worin entweder die vorgenannte erste Verbindung oder die vorgenannte zweite Verbindung oder alle beide ein oder mehrere organische Füllmitel enthalten, die aus einer Gruppe ausgewählt sind, die aus Mikrokugelchen, Quarzpartikeln, Korundpartikeln, Mikrokiesel, Rauchkiesel besteht.

11. Verbindung nach Anspruch 10, worin der vorgenannte erste Bestandteil ein Gemisch von Poly(Methyl-Methakrilat) und Methyl-Methakrylat, eine Mischung von ätherischen Ölen mit Minzöl, Eukalyptusöl und Bergamotöl und einen Polymerisation-Initiator enthält und der vorgenannte zweite Bestandteil Benzoyl-Peroxide, Glass-Mikrokugelchen und Kiesel-Mikropartikeln enthält.

12. Verbindung nach Anspruch 10, worin der vorgenannte erste Bestandteil Methyl-Methacrylate, Benzalkonium-Chlorid eine Mischung von ätherischen Ölen mit Minzöl, Eukalyptusöl und Bergamotöl, ein Vernetzungsmittel und einen Polymisierungsinitiator enthält und der vorgenannte zweite Bestandteil Poly(Methyl-Methacrylat), Benzoyl-Peroxid, Glass-Mikrokügelchen und Quarz- oder Korundpartikeln enthält.

13. Verbindung nach Anspruch 10, worin der vorgenannte erste Bestandteil eine Mischung von ungesättigten und gesättigten Karbonsäuren mit Diolen oder eine Mischung von flüssigen Epoxyharzen mit Akrylsäure, Benzalkonium-Chlorid, eine Mischung von ätherischen Ölen mit Minzöl, Eukalyptusöl und Bergamotöl, Glass-Mikrokugelchen, Kiesel-Mikropartikeln, ein Vernetzungsmittel und einen Polymerisation-Initiator enthält und der vorgenannte zweite Bestandteil Methyl-Ethyl-Keton-Peroxid enthält.

14. Verbindung nach Anspruch 10, worin der vorgenannte erste Bestandteil eine Mischung von BISGMA (d.h. Bisphenol-Glycidyl-Methacrylat) und TEGDMA (d.h. Triethyllen-Glykol-Dimethacrylat), Benzalkoniumchlorid, eine Mischung von ätherischen Ölen, mit Minzöl, Eukaliptusöl und Bergamotöl, Quarz- oder Korundpartikeln, Glass-Mikrokugelchen und Rauchkiesel und einen Polymerisation- Initiator enthält und der vorgenannte zweite Bestandteil eine Mischung von BISGMA (d.h. Bisphenol-Glycidyl-Methacrylat) und TEGDMA (d.h. Triethylen-Glykol-Dimethacrylat), Benzoylperoxid, Quarz- oder Korundpartikeln, Glass-Mikrokugelchen und Rauchkiesel enthält.

15. Verbindung zur Herstellung von Dentalteilen auf basis von Kunstharzen, wobei die vorgenannte Verbidung der Einzel-Bestandteil-Art ist, worin der vorgenannte Einzel-Bestandteil ein polymerisierbares Kunststoffprodukt und ein Initiator- System enthält, dadurch gekennzeichnet, dass der vorgenannte Einzel-Bestandteil auch eine Quaternär-Ammonium-Verbindung, die eine keimtötende und/oder bakteriostatische Wirkung aufweist, und eine Mischung von ätherischen Ölen, mit Minzöl, Eukalyptusöl und Bergamotöl enthaltenden enthält.

16. Verbindung nach Anspruch 15, worin das vorgenannte polymerisierbare Kunststoffprodukt aus einer Gruppe ausgewält ist, die aus Akryl- und/oder Methacrylverbindungen, BISGMA (d.h. Bisphenol-Glycidyl-Methacrylat) und deren Mischungen mit anderen Dimethacrylaten besteht.

17. Verbindung nach je einem der Ansprüche 15-16, worin die vorgenannte Quaternärammoniumverbindung Benzalkonium-Chlorid ist.

18. Verbindung nach je einem der Ansprüche 15-16, worin der vorgenannte Einzal-Bestandteil auch ein oder mehrere Füllmittel enthält, die aus einer Gruppe ausgewählt sind, die aus Glass-Mikrokugelchen, Quarzpartikeln, Korundpartikeln, mikronisiertem Kiesel, Rauchkiesel besteht.

19. Verbndung nach Anspruch 18, worin der vorgenannte Einzel-Bestandteil eine Mischung von BISGMA (d.h. Bisphenol-Glycidylmethacrylat) und TEGDMA (d.h. Triethylen-Glykol-Dimethacrylat), Benzalkonium-Chlorid, eine Mischung von ätherischen Ölen, mit Minzöl, Eukalyptusöl und Bergamotöl enthaltenden, Quarz- oder Korundpartikeln, Glass-Mikrokugelchen, Rauchkiesel und ein Photoinitiatorsystem enthält.

## Revendications

1. Composition dentaire à base de résines synthétiques, caractérisé en ce qu'elle comprend aussi un compose d'ammonium quaternaire ayant une activité germicide et/ou bactériostatique et un mélange de huiles essentielles comprenant des huiles de menthe, d'eucalyptus et de bergamote.

2. Composition selon la revendication 1, dans laquelle ladite résine synthétique est choisie du group consistant des polymères ou copolymères acryliques, résines des polyesters non saturés, résines d'ester de vinyle, polymères ou copolymères de BISGMA (Bisphénole-glycidyl-méthacrylate).

3. Composition selon la revendication 2, dans laquelle ledit polymère ou copolymère acrylique est un polymère à base de poly (méthyle-méthacrylate).

4. Composition selon la revendication 2, dans laquelle ledit polymère ou copolymère de BISGMA est un copolymère de BISGMA et TEGDMA (triéthylène glycol diméthacrylate).

5. Composition selon l'une quelconque des revendications 1-4, dans laquelle ledit composé d'ammonium quaternaire est chlorure de benzalkonium.

6. Composition selon l'une quelconque des revendications 1-5, comprenant aussi un ou plusieurs fillers choisis du group consistant de micro-sphères de verre, particules de quartz, particules de corindon, silice micronisé, silice enfumé.

7. Composition pour la production d'éléments dentaires à base de résines synthétiques, ladite composition étant du type à deux composants, dans laquelle le premier composant comprend un produit synthétique polymérisable et le deuxième composant comprend un initiateur de polymérisation à radicaux libres, caracterisée en ce que ledit premier composant comprend un composé d'ammonium quaternaire ayant une activité germicide et bactériostatique et un mélange de huiles essentielles comprenant des huiles de menthe, d'eucalyptus et de bergamote.

8. Composition selon la revendication 7, dans laquelle ledit produit synthétique polymérisable est choisi du group consistant des composés acryliques et/ou méthacryliques, des mélanges d'acides carboxyliques non saturés et saturés avec diols, des mélanges de résines époxydes liquides avec acide acrylique, BISGMA (bisphénol glycidyl méthacrylate) et leur mélanges avec des autres diméthacrylates.

9. Composition selon l'une quelconque des revendications 7-8, dans laquelle ledit composé d'ammonium quaternaire est chlorure de benzalkonium.

10. Composition selon l'une quelconque des revendications 7-9, dans laquelle ledit premier composant ou ledit deuxième composant ou tous les deux comprendent aussi un ou plusieurs fillers inorganiques choisis du groupe consistant de micro-sphères de verre, particules de quartz, particules de corindon, silice micronisé, silice enfumé.

11. Composition selon la revendication 10, dans laquelle ledit premier composant comprend un mélange de poly(méthyle-méthacrylate) et méthyle-méthacrylate, chlorure de benzalkonium, un mélange d'huiles essentielles comprenant l'huile de menthe, d'eucalyptus et l'huile de bergamote et un activateur de polymérisation, et ledit deuxième composant comprend poly(méthyle-méthacrylate), peroxide de benzoyle, micro-sphères de verre, particules de quartz et de corindon.

12. Composition selon la revendication 10, dans laquelle ledit premier composant comprend méthile-méthacrylate, chlorure de benzalkonium, un mélange d'huiles essentielles comprenant l'huile de menthe, d'eucalyptus et de bergamote, un agent de réticulation et un activateur de polymérization, et ledit deuxième composant comprend poly(méthyle-méthacrylate), peroxide de benzoyle, micro-sphères de verre et particules de quartz et de corindon.

13. Composition selon la revendication 10, dans laquelle ledit premier composant comprend un mélange d'acides carboxyliques non saturés ou saturés avec diols ou un mélange de résines époxydes liquides avec acide acrylique, chlorure de benzalkonium, un mélange d'huiles essentielles comprenant l'huile de menthe, d'eucalyptus et de bergamote, silice micronisé, un agent de réticulation et un activateur de polymérisation et ledit deuxième composant comprend peroxyde de méthyle-éthylecétone.

14. Composition selon la revendication 10, dans laquelle ledit premier composant comprend un mélange de BISGMA (bisphénole-glycidyle-méthacrylate) et TEGDMA (triéthilene-glycole-diméthacrylate), chlorure de benzalkonium, un mélange d'huiles essentialles comprenant l'huile de menthe, l'huile d'eucalyptus et l'huile de bergamote, des particules de quartz au de corindon, micro-sphéres de verre, silice enfumé et un activateur de polymérisation, et ledit deuxième composant comprend un mélange de BISGMA (bisphénole-glycidyle-méthacrylate) et TEGMA (triéthylene-glycole-diméthacrylate), peroxyde de benzoyle, des particules de quartz ou de corindon, micro-sphéres de verre et silice enfumé.

15. Composition pour la production d'éléments dentaires à base de résines synthétiques, ladite composition étant du type à composant unique, dans laquelle ledit composant unique comprend un produit synthétique polymérisable et un système de photo-initiateur, caractérisé en ce que ledit composant unique comprend un composé d'ammonium quaternaire ayant une activité germicide et bactériostatique, et un mélange d'huiles essentielles comprenant l'huile de menthe, l'huile d'eucalyptus et l'huile de bergamote.

16. Composition selon la revendication 15, dans laquelle ledit produit synthétique polymérisable est choisi du groupe consistant des composés acryliques et/ou méthacryliques, BISGMA (bisphénole-glycidyle-méthacrylate) et leur mélanges avec des autes diméthacrylates.

17. Composition selon l'une quelconque des revendications 15-16, dans laquelle ledit composé d'ammonium quarternaire est chlorure de benzalkonium.

18. Composition selon l'une quelconque des revendications 15-17, dans laquelle ledit composant unique comprend aussi un ou plusieurs fillers inorganiques choisis du groupe consistant des micro-sphéres de verre, particules de quartz, particules de corindon, silice micronisé, silice enfume.

19. Composition selon la revendication 18, dans laquelle ledit composant unique comprend un mélange de BISGMA (bisphénole-glycidyle-méthacrylate) et TEGDMA (triéthylene-glycole-diméthacrylate), chlorure de benzalkonium, un mélange des huiles essentielles comprenant l'huil de menthe, l'huil d'eucalyptus et l'huil de bergamote, des particules de quartz ou de corindon, des micro-sphéres de verre, silice enfumé et un système de photo-initiateur.
